# EUROPÄISCHE PATENTSCHRIFT

(11) **EP 1 061 354 B1**
(45) Veröffentlichungstag und Bekanntmachung des Hinweises auf die Patenterteilung: **02.05.2007**
(21) Anmeldenummer: 00111245.7
(22) Anmeldetag: 25.05.2000
(51) Int. Cl.: G01N 3/42, G01N 33/44, G01N 3/30

(54) **Verfahren zur Ermittlung der Haptik von weichen Materialien und Vorrichtung zur Durchführung des Verfahrens**
Method and device for determining tactile qualities of soft materials
Méthode et dispositif pour la détermination des qualités tactiles de matériaux souples

(30) Priorität: 17.06.1999 DE 19927644
(43) Veröffentlichungstag der Anmeldung: 20.12.2000
(73) Patentinhaber: Bayerische Motoren Werke Aktiengesellschaft, 80809 München (DE)
(72) Erfinder: Schmitt, Josef, 84130 Dingolfing (DE); Busler, Max, 94428 Eichendorf (DE); Artmann, Johann, 84160 Frontenhausen (DE); Messerschmidt, Rudolf, 84180 Kronwieden (DE)

(56) Entgegenhaltungen:
- EP-A- 0 457 412
- EP-A- 0 867 708
- DE-A- 2 809 227
- DE-A- 3 830 815
- DE-A- 4 021 178
- DE-A- 19 521 427
- DE-C- 19 516 643
- DE-C- 19 609 881
- US-A- 4 383 450
- US-A- 4 450 713
- LANDMANN A W ET AL: "Softness - an international comparison of manual assessment against instrumental methods" JOURNAL OF THE SOCIETY OF LEATHER TECHNOLOGISTS & CHEMISTS, MAY-JUNE 1994, SOC OF LEATHER TECHNOLOGISTS AND CHEMISTS, MOULTON, ENGAND, Bd. 78, Nr. 3, Mai 1994 (1994-05), Seiten 88-92, XP002220894

## Beschreibung

Die Erfindung bezieht sich auf ein Verfahren zur Ermittlung der Haptik von weichen Materialien, insbesondere Leder, und eine Vorrichtung zu ihrer Durchführung.

Eine Vorrichtung zur Bestimmung der Härte und anderer Eigenschaften von Stoffen wie Metallen, Kunststoffe, Gummis u.a. ist aus der Patentschrift DE 195 16 643 bekannt. Diese offenbart einen von einer Antriebseinheit auf die Materialprobe gedrückten Prüfkörper, der mit einem Wegaufnehmer für den Vorschubweg und mit einem Kraftaufnehmer ausgestattet ist, wobei mit Hilfe einer Auswerteeinrichtung Kraft-Weg-, Kraft-Zeit- und Weg-Zeit- Diagramme erhalten werden.

Aus dem DIN-Norm-Entwurf prEN 13338 zur Bestimmung der Weichheit von Leder ist es bekannt, die Lederoberfläche mittels eines massebelasteten Prüfstiftes zu belasten, wobei in Abhängigkeit der Lederqualität die Größe der Lederprobe gewählt wird. Die aufgrund der definierten Masse erzeugte Ausdehnung des Leders wird mit einer Meßuhr gemessen.

Nachteilig bei diesem bekannten Verfahren ist es, daß keine genauen und reproduzierbaren Ergebnisse erzielt werden. Die Ausgangsparameter wie Eindringkraft und Eindringtiefe werden nicht ausreichen konstant gehalten. Auch wird weder die Dicke der Lederprobe noch ihre Zurichtungsbeschaffenheit bei der Prüfung vorgegeben.

Deshalb ist es allgemein üblich, das Leder mit Hand durch erfahrene Fachleute zu prüfen und in die bekannten Qualitätsstufen einzuteilen.

Dieses Verfahren ist jedoch sehr subjektiv, so daß es immer wieder bei einer Weiterverarbeitung des Leders, z.B. beim Hinterschäumen, zu Fehlern kommt, die dann nicht mehr reparabel sind. Das so produzierte Bauteil muß dann vernichtet werden.

Aufgabe der vorliegenden Erfindung ist es, ein Verfahren bereitzustellen, mit dem schnell und einfach mit reproduzierbaren Werten die Haptik von weichen Materialien, insbesondere Leder, überprüft werden kann. Weiterhin soll eine Vorrichtung vorgeschlagen werden, mit der eine zerstörungsfreie Prüfung der weichen Materialien durchgeführt werden kann.

Die Aufgabe wird erfindungsgemäß durch die Merkmale des ersten und dritten Anspruchs gelöst.

Der Lösung liegt die Idee zugrunde, daß bei den zu prüfenden Materialien die Eindrücktiefe in Abhängigkeit der Materialstärke vorgenommen wird und daß die hierbei ermittelte Reaktionskraft bis zum Erreichen der vorgegebenen Eindrücktiefe insbesondere in Abhängigkeit der Zeit oder in Abhängigkeit des Eindrückweges bis zur vorgegebenen Eindrücktiefe aufgezeichnet wird. Hiermit können reproduzierbare Werte, insbesondere für Leder, hinsichtlich seiner Weichheit, Flexibilität und Dämpfungseigenschaft ermittelt werden. So wird die Weichheit des Leders anhand der maximalen Reaktionskraft bestimmt, d.h. je höher diese ist, um so härter ist das Leder, da ein höherer Kraftaufwand notwendig war, um die vorgegebene Eindrücktiefe zu erlangen.

Eine Aussage über die Flexibilität kann aus den Kraft-/Weg- bzw. Kraft-/Zeit-Kurvenverläufen bei gleicher oder unterschiedlicher Eindrückgeschwindigkeit ermittelt werden.

Ein Maß für die Dämpfungseigenschaften ist der Verlauf des Kraftabfalles nach dem Erreichen der Endposition. Die entsprechenden Werte lassen sich leicht dadurch erreichen, daß - wie Anspruch 2 vorschlägt - eine elektronische Auswerteeinrichtung vorgesehen ist, mit dem der Kraftverlauf pro Zeiteinheit, die Maximalkraft und der Kraftverlauf pro Wegeinheit aufgezeichnet werden kann. Versuchsreihen, bei denen zuerst fachkundige Testpersonen das Leder beurteilt haben und dann anschließend das Leder gemäß der Erfindung gemessen wurde, haben die Aussagekraft des Meßverfahrens bestätigt. In den Fällen, in denen Abweichungen zwischen dem Eindruck der Testperson und dem Meßergebnis aufgetreten sind, hat die Weiterverarbeitung des Leders das Meßergebnis bestätigt. Solche Fälle können beispielsweise auftreten, wenn die Materialoberfläche vor der Prüfung bearbeitet war, beispielsweise wenn Gleitmittel eingesetzt worden sind oder eine mechanische Bearbeitung, wie Beschleifen und Walken, durchgeführt wurde. In solchen Fällen ermittelten die Testpersonen in der Regel eine höhere Weichheit der Probe als das Meßverfahren, jedoch hat eine spätere Weiterverarbeitung gezeigt, daß das Leder in der Tat wesentlich härter war und es deshalb bei der Weiterverarbeitung in vielen Fällen zu Problemen kam.

Die Ausbildung der Erfindung gemäß Anspruch 3 hat den Vorteil, daß ein universell einsetzbares Prüfgerät zur zerstörungsfreien Vorortprüfung der Haptik von ganzen Lederhäuten oder auch von Lederproben zur Verfügung steht. Somit ist es möglich, die Lieferqualität des Leders zu überwachen, da für die erfindungsgemäße Prüfung keine Proben entnommen werden müssen.

Die Meßreihen können verbessert werden, wenn die ermittelten Meßwerte mit dem Ergebnis des fertigen Produktes und den bei der Fertigung auftretenden Problemen oder dem Fehlen von Problemen verglichen werden. So können aus den Meßreihen auch Ledereigenschaften abgeleitet werden für verschiedene Bearbeitungsprozesse, da die Meßergebnisse selbst immer wieder reproduzierbar einfach und schnell erzeugt werden können.

Die Ermittlung der Eindrücktiefe in Abhängigkeit der Lederdicke geschieht ebenfalls in Testreihen, wobei sich herausgestellt hat, daß Eindrücktiefen von 0,5 bis 0,8 mm bei Lederdicken von 1 bis 1,6 mm ausreichen.

Bisher und im folgenden wurde immer von Leder gesprochen. Selbstverständlich ist das erfindungsgemäße Verfahren und die erfindungsgemäße Vorrichtung nicht auf Leder beschränkt. Verfahren und Vorrichtung eignen sich ebenso zur Prüfung von anderen weichen Materialien wie Gummi, Schaum, Dichtungen etc.

Die Weiterbildung der Erfindung nach den Ansprüchen 4 bis 10 stellen bevorzugte Ausführungsformen der erfindungsgemäßen Vorrichtung dar. Hierbei ist es möglich, den Prüfkörper hydraulisch mit Hilfe einer Zylinderkolbeneinheit anzutreiben oder mit Hilfe eines genau definierten Gewichtes. Der Unterschied in der Eindrückgeschwindigkeit zwischen der Zylinderkolbeneinheit und dem Fallgewicht spielt keine Rolle, wie Versuche ergeben haben.

Im folgenden wird die Erfindung anhand eines ausgewählten Beispieles näher erläutert. Es stellen dar
- Figur 1: eine Prinzipskizze einer ersten Prüfvorrichtung;
- Figur 2: ein Schaltplan für die Antriebseinrichtung gemäß Figur 1;
- Figur 3: eine Prinzipskizze einer zweiten Prüfvorrichtung;
- Figur 4: ein typisches Kraft-/Weg-Diagramm zweier Lederproben, die mit der Vorrichtung gemäß Anspruch 3 erreicht werden;
- Figur 5 a,b: Kraft-/Zeit- und Kraft-/Weg-Diagramme mit unterschiedlichen Belastungszeiten;
- Figur 6 a bis d: Kraft-/Weg- und Kraft-/Zeit-Diagramme für unterschiedliche Lederproben;
- Figur 7 a bis c: Kraft-/Weg- und Kraft-/Zeit-Diagramme einer einzigen Lederprobe mit unterschiedlichen Meßparametern;
- Figur 8 a,b: Kraft-/Weg- und Kraft-/Zeit-Diagramme von Lederproben, die subjektiv als weich empfunden werden.

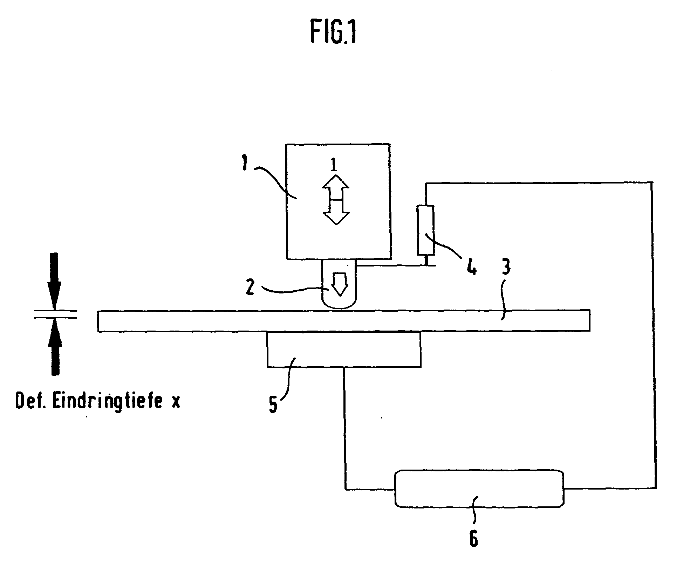

In Figur 1 ist als Prinzipskizze ein erster Prüfaufbau dargestellt, mit dem gemäß der Erfindung die Haptik von weichen Materialien überprüft werden kann. Sie besteht aus einer Antriebseinrichtung 1, die einen Prüfkörper 2 bewegt. Mit 3 ist eine Lederprobe bezeichnet. Ein Wegaufnehmer 4 erfaßt den Weg des Prüfkörpers.

Die Lederprobe 3 liegt auf einem Kraftaufnehmer 5, der unterhalb des Prüfkörpers 2 angeordnet ist.

Sowohl die Ausgangssignale des Wegaufnehmers als auch des Kraftaufnehmers werden zu einer Auswerteeinrichtung 6 geleitet.

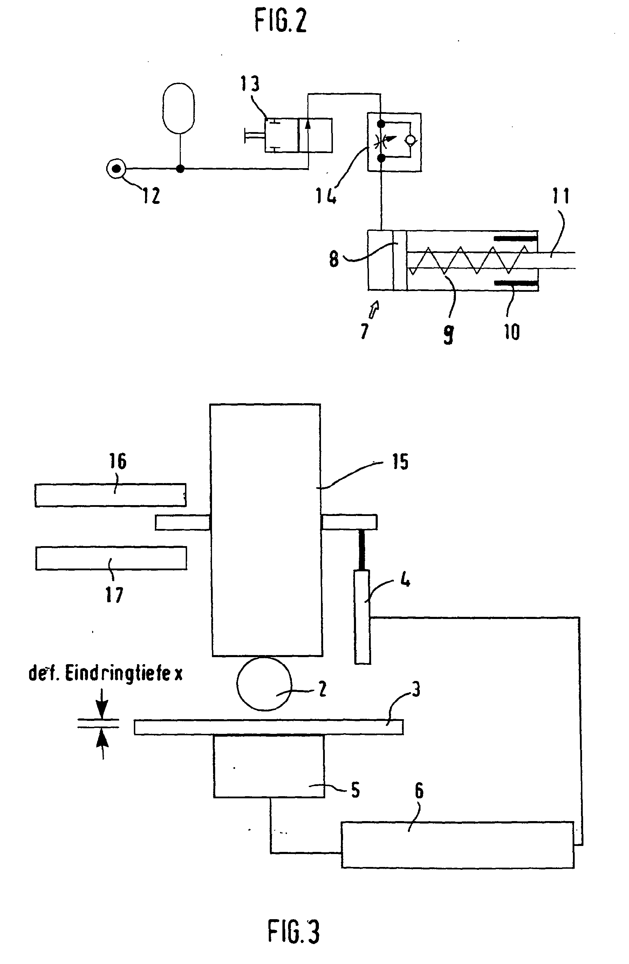

In Figur 2 ist schematisiert ein Schaltplan zum Antrieb des Prüfkörpers 2 dargestellt. Im vorliegenden Beispiel besteht sie aus einer Zylinderkolbeneinrichtung 7, bei der der Kolben 8 gegen die Kraft einer Feder 9 von einem Hydraulikdruck bis zu einem Anschlag 10 verfahren werden kann. Die mit dem Kolben 8 verbundene Kolbenstange 11 trägt den Prüfkörper 2.

Der Hydraulikdruck wird von einer entsprechenden Hydraulikquelle 12 geliefert. Zum Betätigen der Zylinderkolbeneinheit 7 dient das Schaltventil 13. Zum Realisieren unterschiedlicher Verfahrgeschwindigkeiten des Kolbens 8 dient das einstellbare Drosselventil 14.

Zum Messen einer Lederprobe 3 wird diese auf den Kraftaufnehmer 5 gelegt. Aufgrund der Feder 9 befindet sich der Prüfkörper 2 in seiner eingefahrenen Stellung. Als Prüfkörper 2 kann beispielsweise eine Kugel dienen.

Zum Justieren des Wegaufnehmers 4 wird der Prüfkörper 2 bis auf die Lederoberfläche abgesenkt. Der Kontaktdruck wird über die Lederprobe auf den Kraftaufnehmer 5 übertragen und auf der Auswerteeinrichtung 6 angezeigt. Der gemessene Abstand zwischen dem Prüfkörper 2 und der Oberfläche des Kraftaufnehmers 5 entspricht nun exakt der Probendicke an der zu messenden Stelle. Aus dieser Position wird der Prüfkörper in Abhängigkeit der gemessenen Probendicke definiert eingedrückt. Die maximale Eindrücktiefe hängt ab von der Dicke der Lederprobe und wird in experimentellen Reihen ermittelt. Beispielsweise wird ein Eindruckweg von 0,5 bis 0,8 mm verwendet für Lederdicken von 1 bis 1,6 mm.

Beim Eindrücken der Lederprobe 3 wird die Kraft mit Hilfe des darunterliegenden Kraftaufnehmers 5 in Abhängigkeit von der Zeit und dem Weg gemessen. Mit Hilfe des Auswerteeinheit 6 kann dann die gemessene Maximalkraft und in grafischer Darstellung der Kraftaufbau in Abhängigkeit der Zeit und des Weges angezeigt und/oder ausgedruckt werden.

Als Kraftaufnehmer können DMS/Quarze dienen. Als Wegaufnehmer eignen sich Induktionswegaufnehmer oder Potentiometer.

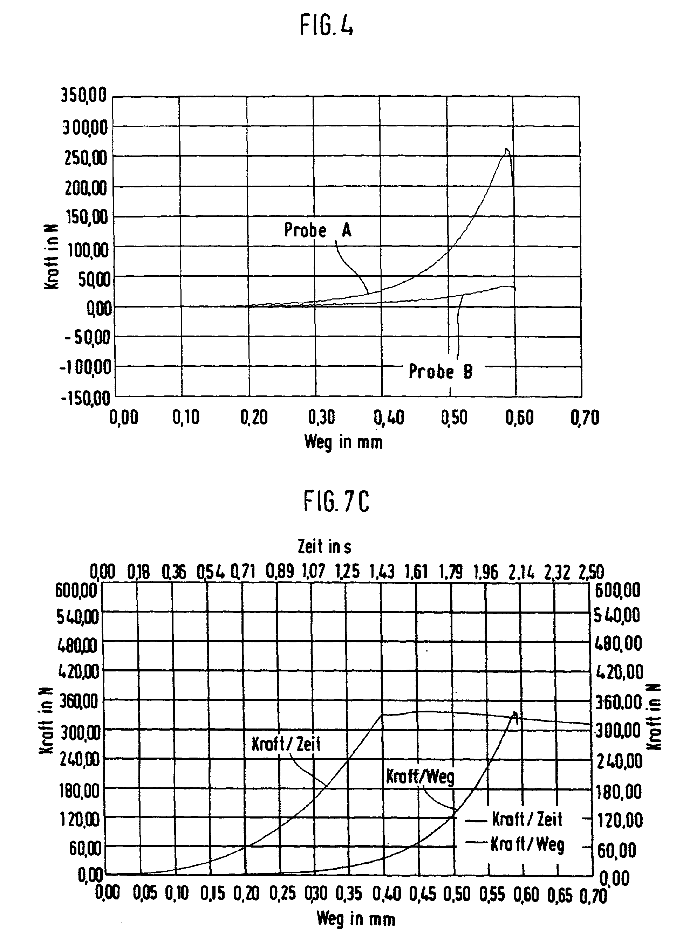

Ein typisches Kraft-/Weg-Diagramm, das mit der Vorrichtung gemäß Anspruch 1, 2 aufgenommen werden kann, zeigt Figur 4. Wie daraus ersichtlich, muß zum Erreichen der vorgegebenen Eindringtiefe, in diesem Beispiel von 0,6 mm, bei der Probe A eine Kraft von 250 N aufgewandt werden, während bei der Probe B nur eine Kraft von ca. 40 N benötigt wird, um die gleiche Eindrücktiefe aufzuweisen. Damit kann die Probe A als hart und die Probe B als weich eingestuft werden.

Bevor auf die weiteren Diagramme eingegangen wird, wird noch auf eine alternativ aufgebaute Vorrichtung nach Figur 3 hingewiesen. Bei diesem Meßgerät wird keine Hydraulikquelle benötigt, sondern das Eindrücken des Prüfkörpers geschieht über ein Fallgewicht 15, das zwischen zwei Anschlägen 16 und 17 bewegt werden kann.

Der Anschlag 16 dient zur Fallhöhenbegrenzung, während der Anschlag 17 die Eindrücktiefe begrenzt. Beide Anschläge 16 und 17 sind höheneinstellbar.

Die übrigen Elemente der Prüfvorrichtung sind identisch aufgebaut wie die Prüfeinrichtung nach Figur 1.

Der Vorteil der Prüfvorrichtung nach Figur 3 besteht darin, daß keine Hydraulikquelle benutzt wird, so daß mit diesem Meßgerät eine zerstörungsfreie Prüfung von Materialproben vor Ort in der Produktion oder auch im Wareneingang vorgenommen werden kann.

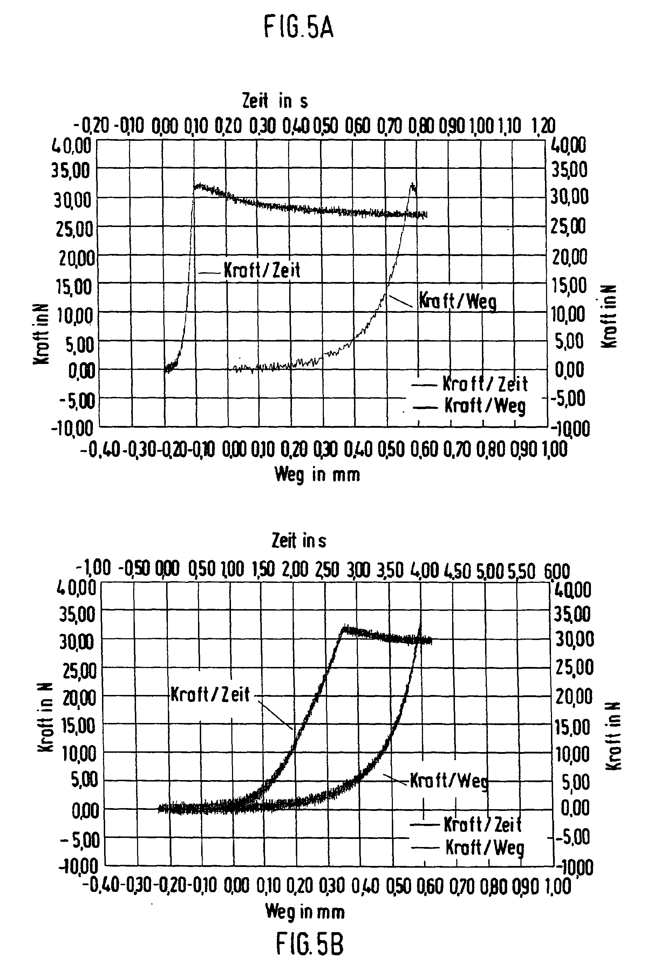

Die Diagramme in Figur 5 a und b stellen Kraft-/Weg- und Kraft-/Zeit-Diagramme einer weichen Lederprobe dar, da die Maximalkraft nur ca. 33 N beträgt. Sie unterscheiden sich in der Höhe der Belastungszeit. In Figur 5 a wurde der Prüfkörper in ca. 0,1 Sekunden auf seine maximale Eindrücktiefe bewegt, während in Figur 5 b hierfür 3 Sekunden benötigt wurden. Man sieht daraus, daß die Eindrückgeschwindigkeit in der Regel keinen nennenswerten Einfluß auf die Meßergebnisse hat. In beiden Fällen wurde nur eine geringe Eindrückkraft benötigt, so daß in beiden Fällen von einem weichen Leder gesprochen werden kann.

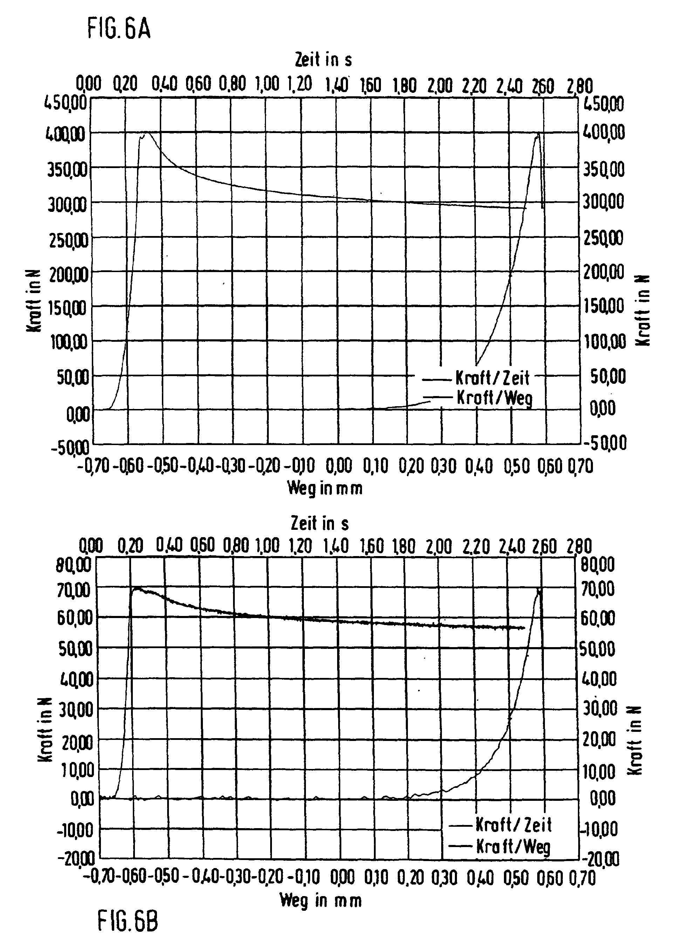

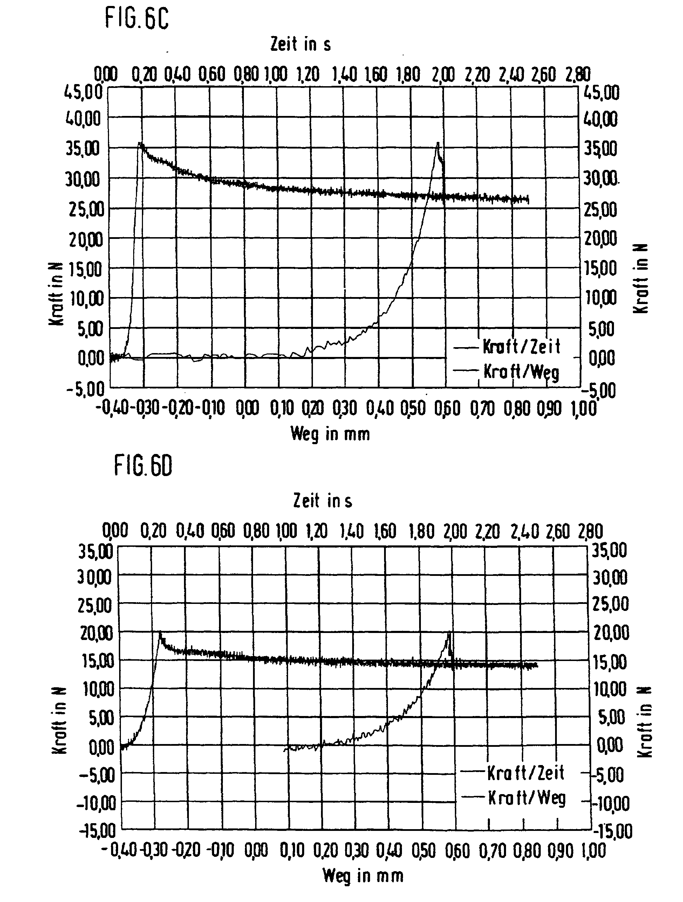

In den Figuren 6 a bis 6 d sind Kraft-/Weg- (rechte Kurve) und Kraft-/Zeit-Diagramme (linke Kurve) verschiedener Lederproben dargestellt, die von den Lederlieferanten als Softleder eingestuft wurden. Diese Einstufung basiert auf dem subjektiven Urteil erfahrener Prüfer.

Wie sich unschwer aus Figur 6 a herleiten läßt, ist dieses Leder extrem hart, trocken und unflexibel. Aufgrund der Maximalkraft von 400 N muß es als sehr hart eingestuft werden. Die hier ermittelten Eigenschaften haben sich bei einer Weiterverarbeitung auch bestätigt.

Wesentlich weicher ist die Lederprobe gemäß Figur 6 b. Dort ist die Maximalkraft nur noch 70 N. Auch weist dieses Leder gute mechanische Eigenschaften hinsichtlich Dehnung und Flexibilität auf. Aufgrund von weiteren Untersuchungen und Weiterverarbeitungsversuchen kann diese Lederprobe als noch weich eingestuft werden.

Die Lederprobe gemäß Figur 6 c zeigt eine Maximalkraft von ungefähr 35 N. Damit kann das Leder als sehr weich und flexibel eingestuft werden, jedoch sind die Dehnwerte, die sich aus mehreren Messungen der Probe und Vergleich der Kurvenverläufe bei gleicher oder unterschiedlicher Eindringgeschwindigkeit ergeben, zu hoch.

Die dem Diagramm in Figur 6 d zugrundeliegende Lederprobe zeichnet sich durch eine sehr hohe Qualität aus. Sie ist die weicheste aller vier Proben mit einem Maximalwert von ca. 20 N. Auch weist sie sehr gute Dehnungswerte und eine hohe Flexibilität auf. Hierbei handelt es sich um naturbelassenes Softleder.

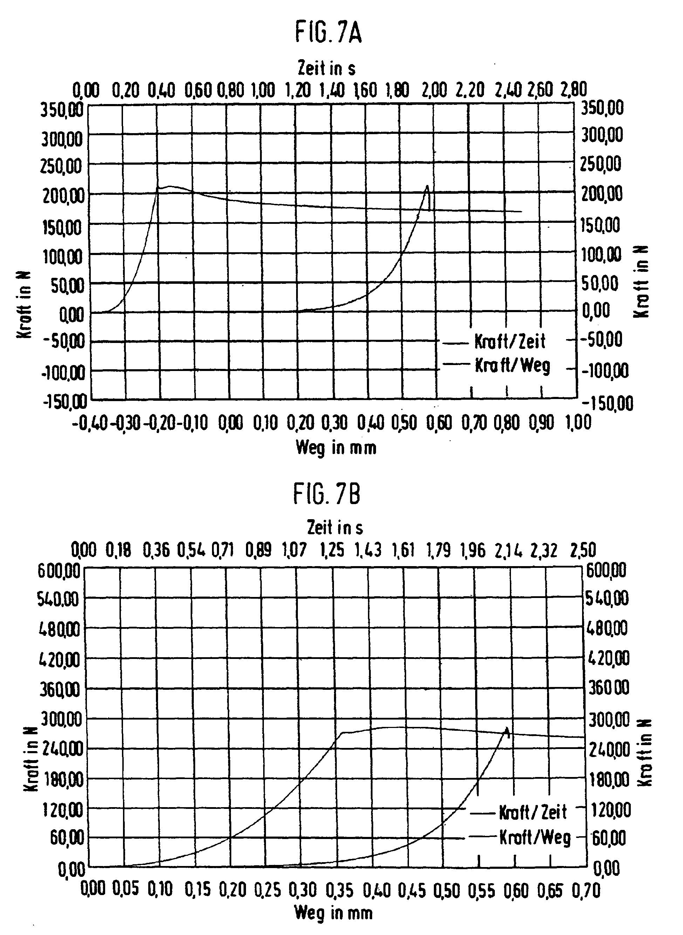

In den in den Figuren 7 a bis 7 c dargestellten Kraft-/Weg- (rechte Kurve) und Kraft-/Zeit- (linke Kurve) Diagrammen wurde eine einzige Lederprobe mit unterschiedlichen Parametern vermessen. Das Diagramm in Figur 7 a zeigt eine Messung bei einer Probendicke von 1,45 mm, einer Eindrücktiefe von 0,6 mm und einer Eindrückzeit von ca. 0,4 Sekunden. Die Maximalkraft liegt bei 220 N, also handelt es sich um eine harte Lederprobe.

In Figur 7 b wird die gleiche Lederprobe ebenfalls 0,6 mm eingedrückt, jedoch ist hier eine längere Eindrückzeit von ca. 1,5 Sekunden angewandt worden. Die Maximalkraft liegt sogar hier noch höher als in dem Diagramm nach Figur 7 a.

In Figur 7 c wurde die Probendicke auf 1,3 mm reduziert. Die Eindrückzeit war länger, ca. 1,6 Sekunden, während die Eindrücktiefe wie in den übrigen Beispielen bei 0,6 mm lag. Hierbei stellte sich eine Maximalkraft von 340 N ein.

Als Ergebnis aus diesen drei Diagrammen kann man festhalten, daß bei hartem Leder die Differenz der gemessenen einzelnen Reaktionskraft des Leders bei unterschiedlichen Eindrücktiefen höher ist als bei weichem Leder. Der Einfluß der Probendicke ist also besonders zu beachten.

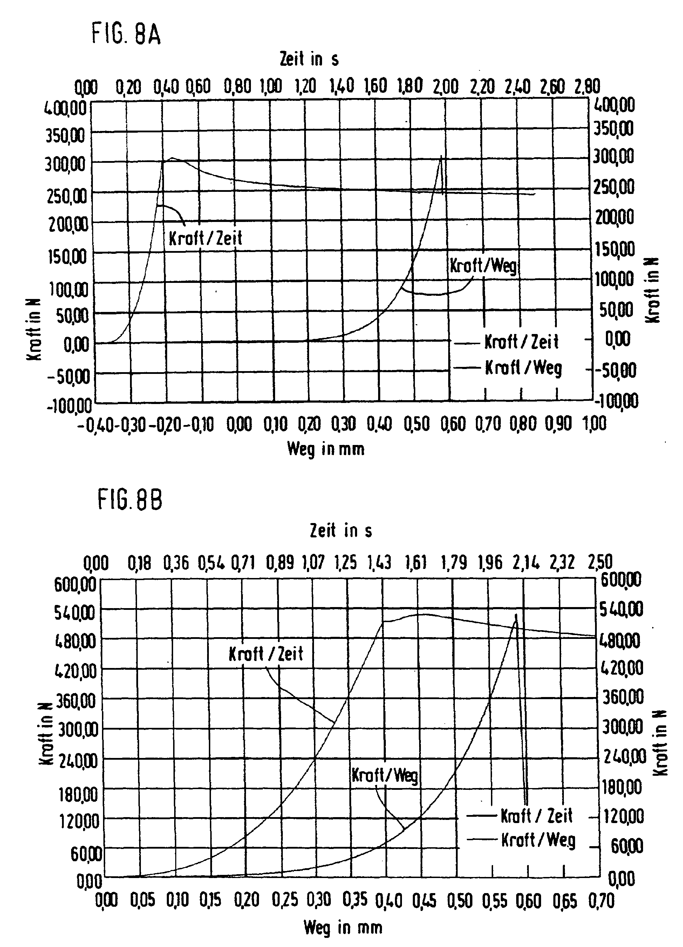

In den beiden Diagrammen in Figur 8 a und 8 b wurde ein als subjektiv weich und flexibel eingestuftes Leder mit dem erfindungsgemäßen Meßgerät untersucht. Hierbei wird deutlich, daß die Meßwerte erheblich von dem subjektiven Eindruck abweichen. Gemäß den Meßwerten handelt es sich um ein hartes Leder. Der subjektive Eindruck kann durch Bearbeiten der Narbenseite, durch Zugabe von Gleitmitteln und/oder einer mechanischen Nachbehandlung wie millen, walken, stollen über einen sehr langen Zeitraum von bis zu 5 Stunden hervorgerufen werden. Wie die Versuchsergebnisse zeigen, bewirken diese Behandlungen nur eine Täuschung des subjektiven Eindruckes, während die Meßwerte objektiv eine hohe Maximalkraft anzeigen. Nachfolgende Verarbeitungsversuche haben die Meßwerte bestätigt.

Der Unterschied zwischen den beiden Diagrammen in Figur 8 a und 8 b liegt in der geänderten Eindrückzeit. In Figur 8 a wurde mit ca. 0,4 Sekunden Eindruckzeit gearbeitet, während sie bei Figur 8 b bei 1,6 lag.

## Patentansprüche

1. Verfahren zur Ermittlung der Haptik von weichen Materialien, insbesondere Leder, bei dem ein Prüfkörper (2) senkrecht auf die Materialoberfläche gedrückt wird,
**dadurch gekennzeichnet, dass** der Prüfkörper (2) mit einer vorgegebenen Eindrücktiefe, deren Höhe abhängig ist von der Materialstärke, auf die Materialoberfläche gedrückt wird und das bis zur vorgegebenen Eindrücktiefe die auf der dem Prüfkörper (2) abgewandten Materialoberfläche durchgeleitete Kraft gemessen wird.

2. Verfahren nach Anspruch 1,
**dadurch gekennzeichnet, dass** eine Auswerteeinrichtung (6) vorgesehen ist, die den Kraftverlauf pro Zeiteinheit, die Maximalkraft und den Kraftverlauf pro Weg aufzeichnet.

3. Vorrichtung zur Durchführung des Verfahrens nach einem der Ansprüche 1 oder 2, bestehend aus einer Antriebseinrichtung (1) und einem Prüfkörper (2), die miteinander in Wirkverbindung stehen, und aus einer Aufnahme für das zu prüfende Material (3),
wobei ein Wegaufnehmer (4) für den vom Prüfkörper (2) zurückgelegten Weg vorhanden ist, **dadurch gekennzeichnet, dass** als Aufnahme ein Kraftaufnehmer (5) verwendet wird, der mit dem Wegaufnehmer (4) an eine Auswerteeinrichtung (6) anschließbar ist.

4. Vorrichtung nach Anspruch 3,
**dadurch gekennzeichnet, dass** der Prüfkörper (2) eine Kugel ist.

5. Vorrichtung nach Anspruch 4,
**dadurch gekennzeichnet, dass** die Kugel einen Durchmesser von 10 mm aufweist.

6. Vorrichtung nach einem der Ansprüche 3 bis 5,
**dadurch gekennzeichnet, dass** der Prüfkörper (2) von einer hydraulisch antreibbaren Zylinder-Kolben-Einheit (7) bewegt wird.

7. Vorrichtung nach Anspruch 6,
**dadurch gekennzeichnet, dass** die Zylinder-Kolben-Einheit (7) eine Wegbegrenzung aufweist.

8. Vorrichtung nach einem der Ansprüche 3 bis 5,
**dadurch gekennzeichnet, dass** der Prüfkörper (2) von einem Gewicht (Fallgewicht 15) bewegt wird.

9. Vorrichtung nach Anspruch 8,
**dadurch gekennzeichnet, dass** die Fallhöhe des Gewichtes (Fallgewicht 15) über einen Anschlag (16) einstellbar ist.

10. Vorrichtung nach Anspruch 8 oder 9,
**dadurch gekennzeichnet, dass** die Falltiefe des Gewichtes (Fallgewicht 15) über einen Anschlag (17) einstellbar ist.

## Claims

1. A method of determining the feel of soft materials, especially leather, wherein an indenter (2) is pressed vertically on to the surface of the material,
**characterised in that** the indenter (2) is pressed on to the surface down to a defined depth depending on the thickness of the material, and the force transmitted to the surface of the material remote from the indenter (2) is measured until the defined depth is reached.

2. A method according to claim 1,
**characterised in that** an evaluating device (6) is provided for recording the variation in force per unit time, the maximum force and the variation of force per distance travelled.

3. A device for working the method according to claim 1 or claim 2, comprising a drive device (1), a test member (2) operatively connected thereto, and a pick-up for the material (3) under test, wherein a transducer (4) is provided for the distance travelled by the indenter (2),
**characterised in that** the pick-up is a force transducer (5) which, together with the travel transducer (4), is connected to an evaluating device (6).

4. A device according to claim 3,
**characterised in that** the indenter (2) is spherical.

5. A device according to claim 4,
**characterised in that** the sphere has a diameter of 10 mm.

6. A device according to any of claims 3 to 5,
**characterised in that** the indenter (2) is moved by a hydraulically driven cylinder-piston unit (7).

7. A device according to claim 6,
**characterised in that** the cylinder-piston unit (7) has a travel limiter.

8. A device according to any of claims 3 to 5,
**characterised in that** the indenter (2) is moved by a weight (drop weight 15).

9. A device according to claim 8,
**characterised in that** the drop height of the weight (drop weight 15) is adjustable by means of a stop (16).

10. A device according to claim 8 or claim 9,
**characterised in that** the drop depth of the weight (drop weight 15) is adjustable by means of a stop (17).

## Revendications

1. Procédé permettant de déterminer les propriétés tactiles de matériaux souples, notamment du cuir, selon lequel on presse un corps d'essai (2) perpendiculairement à la surface du matériau,
**caractérisé en ce que**
l'on presse le corps d'essai (2) sur la surface du matériau avec une profondeur de pénétration prédéterminée, dont la hauteur dépend de l'épaisseur du matériau, et on mesure la force transmise à la surface du matériau située à l'opposé du corps d'essai (2) jusqu'à ce que la profondeur de pénétration prédéterminée soit atteinte.

2. Procédé selon la revendication 1,
**caractérisé en ce qu'**
on prévoit un dispositif d'évaluation, qui enregistre la courbe de la force par unité de temps, la force maximale et la courbe de la force par unité de course.

3. Dispositif pour la mise en oeuvre du procédé selon l'une quelconque des revendications 1 ou 2, composé d'un système d'entraînement (1) et d'un corps d'essai (2) en liaison active l'un avec l'autre, et d'un logement pour le matériau à examiner (3), dans lequel on prévoit un enregistreur de course (4) pour la course parcourue par le corps d'essai (2),
**caractérisé en ce qu'**
on utilise comme logement un enregistreur de force (5), qui peut être raccordé avec l'enregistreur de course (4) à un dispositif d'évaluation (6).

4. Dispositif selon la revendication 3,
**caractérisé en ce que**
le corps d'essai (2) est une bille.

5. Dispositif selon la revendication 4,
**caractérisé en ce que**
la bille a un diamètre de 10 mm.

6. Dispositif selon l'une des revendications 3 à 5,
**caractérisé en ce que**
le corps d'essai (2) est déplacé par une unité piston-cylindre (7) à commande hydraulique.

7. Dispositif selon la revendication 6,
**caractérisé en ce que**
l'unité piston-cylindre (7) comprend un limiteur de course.

8. Dispositif selon l'une des revendications 3 à 5,
**caractérisé en ce que**
le corps d'essai (2) est déplacé par un poids (masse tombante 15).

9. Dispositif selon la revendication 8,
**caractérisé en ce que**
la hauteur de chute du poids (masse tombante 15) est réglable par une butée (16).

10. Dispositif selon la revendication 8 ou 9,
**caractérisé en ce que**
la profondeur de chute du poids (masse tombante 15) est limitée par une butée (17).
